(19)

Europäisches Patentamt
European Patent Office
Office européen des brevets

(11) **EP 3 609 947 B1**

(12) **EUROPEAN PATENT SPECIFICATION**

(45) Date of publication and mention
of the grant of the patent:
**29.03.2023 Bulletin 2023/13**

(21) Application number: **18721598.3**

(22) Date of filing: **12.04.2018**

(51) International Patent Classification (IPC):
**C08G 69/28** (2006.01)    **B01J 19/00** (2006.01)
**G01N 21/65** (2006.01)

(52) Cooperative Patent Classification (CPC):
**C08G 69/28; B01J 4/008; B01J 19/0006;
G01N 21/65;** G01N 2201/129

(86) International application number:
**PCT/US2018/027213**

(87) International publication number:
**WO 2018/191445 (18.10.2018 Gazette 2018/42)**

(54) **MONOMERIC BALANCE CONTROL IN THE PREPARATION OF PRECURSORS FOR
POLYAMIDATION PROCESSES**

MONOMERE BALANCESTEUERUNG BEI DER HERSTELLUNG VON VORLÄUFERN FÜR
POLYAMIDIERUNGSVERFAHREN

RÉGULATION D'ÉQUILIBRE MONOMÉRIQUE DANS LA PRÉPARATION DE PRÉCURSEURS
DESTINÉS À DES PROCÉDÉS DE POLYAMIDATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **13.04.2017   US 201762485058 P**

(43) Date of publication of application:
**19.02.2020   Bulletin 2020/08**

(73) Proprietor: **INVISTA Textiles (U.K.) Limited
Manchester M2 3DE (GB)**

(72) Inventors:
• **KAUSHIVA, Bryan Dinesh
Wakefield
Yorkshire WF2 6PA (GB)**
• **MONSTER, Leen
3214 TD Zuidland (NL)**

(74) Representative: **Carpmaels & Ransford LLP
One Southampton Row
London WC1B 5HA (GB)**

(56) References cited:
**WO-A1-2013/085747    US-A1- 2004 083 028**

EP 3 609 947 B1

**Description**

FIELD

This disclosure relates to a method for preparing aqueous salt solutions suitable as feeds for polyamidation. Raman spectroscopy and chemometric modelling are applied to provide an estimate of molar balance that is useful in characterization and control of either continuous, batch or semi-batch processes or feed systems used to supply polyamidation processes with suitable precursors.

BACKGROUND

Successful production of polyamides requires precise control of the starting monomeric balance of the salt solutions. Generally, this is achieved by first making an approximately balanced solution of the starting materials followed by one or more adjustment steps. These processes may be conducted in continuous, semi-batch or batch-wise process schemes.

The preparation of aqueous solutions containing diamines and dicarboxylic acids is an important and common intermediate step in their conversion into useful polyamides. Industrial processes making these solutions require control schemes that are affordable and capable of maintaining the process streams at their desired composition and concentration. Effective control improves polymer quality and avoids undesirable precipitation of solids. Several approaches are suitable for approximate control of initial steps of a preparation. Control based only on feed rates to the first mixing steps can be accomplished with volumetric, gravimetric or other conventional process instrumentation. Online pH instruments are also available. Best results with pH based systems require extensive control of dilution and temperature for the side stream to be analyzed by the pH instrument.

Previously known salt production facilities generally depend upon pH instrumentation as a basis of control. pH instruments have limited precision due to their accuracy being determined by the buffer solutions used to calibrate them. In addition, the pH instruments are subject to drift and fouling. The accuracy of the pH measurement is such that it requires salt storage and blending to reduce salt balance variability to prevent swings of salt properties for the salt being used for polymer production. Storing salt is expensive and limits the maximum concentration of the salt used.

Fine tuning the monomeric balance may be achieved within semi-batch or batch-wise operations by iterative steps of collecting samples for laboratory pH testing followed by adjustments in the vessels.

US 3,730,685 relates to a process of automatically performing titrations of solutions.

US 4,213,884 and US 4,251,653 relate to a two-step process for the production of concentrated salt solutions.

US 4,442,260 relates to a multi-step process for the production of concentrated aqueous salt solutions using weight of salt charge as a key process control parameter.

US 5,801,278 relates to a method of directly preparing salts in substantially solid particulate form in which variation in pH is used as an indicator of process consistency.

US 9,561,999 relates to a two-step salt process using measurement of pH for control of salt monomeric balance.

Respective aspects of a group of recent applications may be considered together to illustrate the relative complexity of current control systems: WO 2014/179057, WO 2014/179058, WO 2014/179060, WO 2014/179061, WO 2014/179062, WO 2014/179064, WO 2014/179065, and WO 2014/179066. The references relate to various features including weight-in-loss feeders to limit feed rate variability of the powdered dicarboxylic acid; sample loops for dilution and temperature control for online pH measurements; offline pH measurements; sample loops for the measurement of concentration using refractometers; density measurement; and the use of the various measurements from the systems described to build predictive models to tune control of molar balance and concentration.

US 8,772,439 relates to the two-step processes of earlier patents to produce aqueous salt solutions. The diacid-diamine balance is determined by pH.

US 8,802,810 relates to multi-step process to produce aqueous salt solutions of varying combinations of aliphatic, cycloaliphatic and aromatic diacids. Molar balance between the diacids and diamines is based upon pH.

US 9,561,999 relates to the sensitivity of pH to temperature, composition and concentration.

US 4,233,234 relates to a continuous preparation of aqueous nylon salt solutions in which a small fraction of the bulk stream is then taken off, diluted to 10% concentration and the pH is measured. The pH signal is used to control the amount of trim diamine injected into the bulk stream as required to tune the molar balance.

US 2,130,947A relates to the inflection of the pH curve as the control point for ensuring the diamine and dicarboxylic acid are at a molar ratio that will polymerize to high molecular weight.

Another technique in the art to measuring salt balance is that of near infrared (NIR) spectroscopy. See for example, US 5,532,487, US 6,169,162, US 6,696,544 and US 6,995,233. NIR has the advantage of using a rugged online probe. However, the instruments can be challenging to calibrate and the calibration must be maintained. In addition, the near infrared region is strongly sensitive to moisture content and temperature changes. Unlike with pH systems, the NIR systems do not demand that moisture content and temperature be held fixed; however, to be successful, the NIR processor must have accurate concentration and temperature data as an input to the PLS model. The NIR calibration set must incorporate those variables and the subsequent model must be supplied with reliable and accurate data. These sensitivities are demanding and reduce the robustness of the technique.

The limits of these two approaches can be seen in the designs of the manufacturing sites. To ensure stable and consistent operation of the downstream polymerization facilities, it is common to use large intermediate storage vessels following salt preparation. This helps overcome random error in the instrumentation by averaging out short term fluctuations around a set point. Since storage capacity is expensive, it would be desirable to provide a control method that does not require storing significant volumes of material to balance out short-term fluctuations in composition.

The oldest approach is to withdraw a sample from a batch process for off-line dilution and temperature conditioning, followed by pH measurement and any needed batch adjustments. Newer approaches automate this and typically do so in the context of continuous salt processes wherein a slip stream is diverted with dilution and temperature control followed by an online pH instrument. To minimize drift over time, the laboratory pH measurement results can be compared against the online pH results, and the online instrument can be adjusted accordingly. Such improvements have also been applied to NIR-based systems.

Instrumentation used for monitoring and control of polyamidation systems is both expensive and inadequate. An approach is needed to reduce costs and complexity. Therefore, it would be desirable to provide a relatively simple, lower-cost online method for analyzing polyamidation feed streams that functions reliably, consistently and accurately within the normal ranges of process conditions. Such a simple and reliable method is much desired in polyamide manufacturing. The method also needs to be affordable to implement at commercial scale.

Another analytical method is Raman spectroscopy, as disclosed in U.S. Patent 4,620,284 to Schnell et al. The Schnell et al. patent relates to a method that uses Raman to directly identify components in a mixture. Not all mixtures cannot be readily analyzed by Raman. Sometimes the combination of two molecules in solution generates new peaks not characteristic of either component in solution alone. Some solutions can generate spectral features from: (i) the individual components themselves; (ii) interaction among components in solution; and (iii) the total concentration of components in the solution. The precise role of the listed factors in generating spectral features is not fully understood. In particular, aqueous polyamide salt solutions at different salt balances can generate spectra that appear not to provide sufficient information for determining the salt balance.

Thus, the problem is to develop a real-time, online measurement method for controlling the balance between diacid and diamine in an aqueous salt solution for making polyamides.


SUMMARY


Disclosed is a method using a combination of Raman spectroscopy and chemometric modeling for measuring and/or controlling salt balance in a feed to a condensation polymerization process for making polyamides, especially nylon.

Disclosed is a process control method for measuring and controlling diacid-diamine balance of a polyamide precursor solution comprising:

a) creating a predictive model of Raman spectra for diacid-diamine balancec by:

i) acquiring Raman spectra for a series of polyamide precursor solutions having different known molar ratios of diacid:diamine;
ii) modeling the relationship between the known molar ratios and the acquired Raman spectra;

b) applying the model of (a)(ii) to an observed Raman spectrum for a polyamide precursor solution in which the diacid:diamine balance is unknown; and
c) determining the diacid:diamine balance of the solution (b).


One aspect of the disclosed process control method further comprises comparing the diacid:diamine balance determined in step (c) with a setpoint.

Another aspect of the disclosed process control method further comprises adjusting at least one process variable in response to the comparison of the determined diacid:diamine balance with the setpoint.

In one aspect of the disclosed process control method, the polyamide precursor solution comprises at least one dicarboxylic acid, at least one diamine and water.

In another aspect of the disclosed process control method, the dicarboxylic acid comprises adipic acid.

In one aspect of the disclosed process control method, the diamine comprises hexamethylenediamine (HMD).

In a further aspect of the disclosed process control method, the process variable that is adjusted in step (c) is selected from the group consisting of: flow rate of dicarboxylic acid or of a dicarboxylic acid-rich feed stream, flow rate of diamine or of a diamine-rich feed stream, residence time, and flow rate of additive.

BRIEF DESCRIPTION OF THE DRAWINGS

Figure 1 is a representation of an overlay of the Raman spectra of an adipic acid and an HMD solution, according to an embodiment of Example 1.

Figure 2 is a representation of measured Raman spectra according to the Table 1 compositions of Example 1.

Figure 3 is a representation of the four-component PLS model predictability for the Fig. 2 data and according to Example 1. Per each sample a series of consecutive spectra were recorded, each obtained spectrum was analyzed using the four-component PLS model thus providing an estimated DE value.

Figure 4 is a representation of parity plot between the lab analysis data [on X-axis] and averaged estimated DE values [on Y-axis]. The four-component PLS model is developed from twelve-point data according to Example 1.

Figure 5 is a representation of the analysis predictability using an alternate modeling approach according to Example 1 data.

Figure 6 is a schematic representation of feed forward control arrangement according to an embodiment of Example 4.

Figure 7 is a schematic representation of feed-back control arrangement according to an embodiment of Example 5.

Figure 8 is a schematic representation of feed-back control arrangement according to an embodiment of Example 6.

Figure 9 is a schematic representation of process control arrangement according to an embodiment of Example 7.

DETAILED DESCRIPTION

Disclosed is a process control method for measuring and controlling diacid-diamine balance of a polyamide precursor solution comprising:

a) creating a predictive model of Raman spectra for the value of the characteristic by:

i) acquiring Raman spectra for a series of polyamide precursor solutions having different known molar ratios of diacid:diamine;

ii) modeling the relationship between the known molar ratios and the acquired Raman spectra;

b) applying the model of (a)(ii) to an observed Raman spectrum for a polyamide precursor solution in which the diacid:diamine balance is unknown; and

c) determining the diacid:diamine balance of the solution (b).

The disclosed process control method can comprise comparing the diacid:diamine balance determined in step (c) with a setpoint, and can further comprise adjusting at least one process variable in response to the comparison of the determined diacid:diamine balance with the setpoint.

The polyamide precursor solution can comprise dicarboxylic acid, diamine and water. The dicarboxylic acid can be adipic acid, and the diamine can be hexamethylenediamine.

Further disclosed is a process of preparing nylon-6,6 including feeding the polyamide precursor solution to a batch, semi-batch or continuous polymerizer. The process can be controlled by adjusting one or more of: flow rate of dicarboxylic acid or of a dicarboxylic acid-rich feed stream, flow rate of diamine or of a diamine-rich feed stream, residence time, and flow rate of additive.

The thermal polycondensation of salts of diamines and dicarboxylic acids is one of the most important means of production of polyamides via polymerization (also known as polyamidation). Diamine and dicarboxylic acid components are first reacted under conditions effective to form acid-base neutralization salts and the salts are then subjected to polycondensation conditions to prepare polyamides. In some cases, polyamides may be produced via polymerization of aminocarboxylic acids, aminonitriles, or lactams.

The polyamidation or polymerization process can include steps such as a salt strike step where polyamide (e.g., nylon 6,6) salt solutions are prepared using proper monomeric balance, an evaporation step where some of the water from the salt solution is evaporated off, an autoclave process where the salt solution is placed under heat and pressure for polyamidation, and extrusion/cutting steps where the essentially final polymeric raw materials are formed. Other steps can also be included as understood generally by those skilled in the art.

Disclosed is a method using a combination of Raman spectroscopy and chemometric modelling for measuring and/or controlling salt balance in a feed to a condensation polymerization process for making nylon.

Application of chemometric analysis involves calibration. Calibration models can be developed and used to predict properties of interest based on measured properties of a chemical system, such as pressure, flow, temperature, infrared, Raman, NMR spectra and mass spectra. Examples include the development of multivariate models relating to multi-wavelength spectral responses to analyte concentration. The process involves a calibration data by gathering data from a number of reference samples, including analyte concentrations and salt monomeric balance for each sample and the corresponding spectrum of that sample. Multivariate calibration technique such as partial-least squares regression or principal component regression can be used to construct a mathematical model that relates the multivariate response to the analyte concentration. Such a model can be useful to predict the concentrations of new (unknown) samples.

The present disclosure relates to the application of chemometric modelling and analysis of Raman spectra as a means of online analysis and control of salt composition. Advantageously, Raman spectra have relatively little sensitivity to temperature or moisture fluctuations and so provide a more fundamentally stable signal. The reduced sensitivity to temperature and concentration leads to less complicated deployment configurations.

Raman spectra can be collected at process conditions providing a simpler deployment scenario than pH measurement. Less sensitivity to moisture and temperature provides fewer sources of noise and error than with NIR modelling. Adequate stability enables using plant configurations without large buffering storage between the balancing point and production.

By combining state-of-the-art probes with high quality spectroscopic instruments and chemometric modelling, a system is attained that can provide fast and accurate feedback with suitable precision for the control of monomeric balance. The speed, precision and robustness enables process designs to be considered that are lower cost and can reduce or eliminate storage of balanced salt solutions.

A chemometric model is developed for a specific system by varying the composition of the system and collecting spectra, followed by analysis of the collected samples and analysis of the spectra. The resulting model is then used dynamically by analysing input spectra and obtaining an estimate of the ends balance of the solution.

A variety of commercially available mathematical analysis software packages are well-suited to develop mathematical models of the relationship between the spectra generated by different solutions and the specific concentrations, temperatures, and total concentration of the solutions.

Non-limiting examples of commercially available software packages for chemometric analysis and modeling may include Simca [Sartorius Stedim Biotech; http://umetrics.com/products/simca], PLS Toolbox or Solo [Eigenvector; http://www.eigenvector.corn], The Unscrambler X [Camo Software; http://www.camo.com/rt/Resources/chemometrics.html], GRAMS [ThermoFisher; https://www.thermofisher.com/order/catalog/product/INF-15000], Horizon [ABB; http://new.abb.com/products/measurement-products/analytical/ft-ir-and-ft-nir-software/horizon-mb-qa-software], PLSplus IQ [Adept Scientific; http://www.adeptscience.co.ulc/products/lab/ gramsai/plsplus-iq-spectroscopy-chemometrics-toolbox.html], and like. Any of these software packages can directly accept spectral responses, such as Raman spectra obtained in Example 1, and provide filtering, normalizing, derivatizing or other modifications to support modeling and generation of estimates of underlying characteristics.

Alternatively, the processed data files from the above chemometric modeling may be exported for analysis by other techniques. One example of this approach may be to use peak heights or peak areas of any specific ranges of a spectral response within general multi-variate analysis, such as that illustrated by using MiniTab analysis in Example 3. Several software packages are commercially available to perform such analysis.

The instrumentation probe for Raman spectroscopy measurement may be inserted into a flowline, into a slip stream, into a main process vessel, a transfer or measuring vessel, or into any other suitable process point. The Raman probe may alternatively be located in the lab sample collection point. The choice of probe location must be appropriate for the specific process design and control scheme utilized.

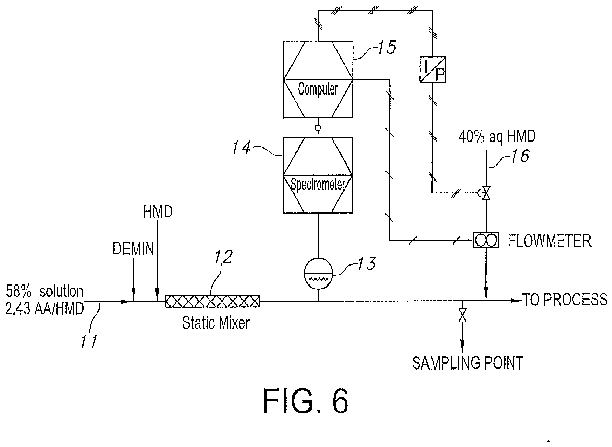

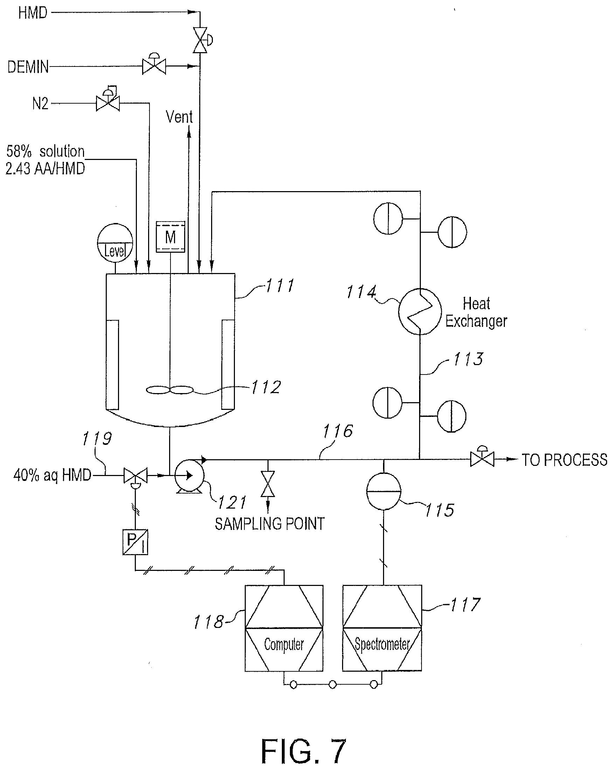

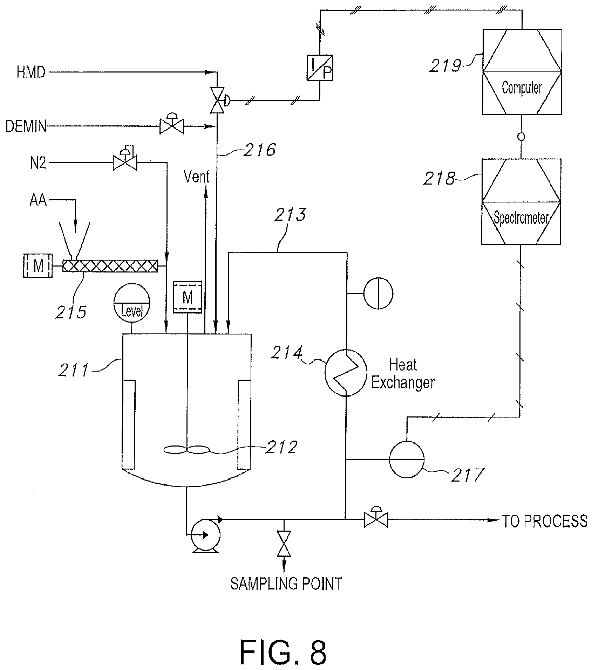

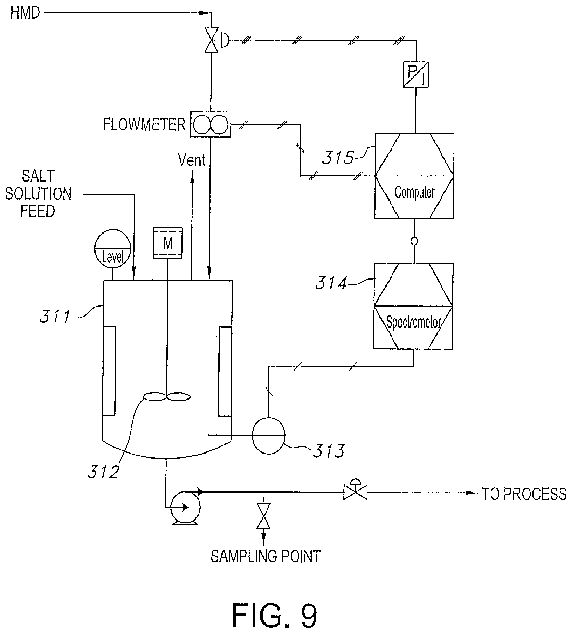

In an embodiment as described in Example 4 and schematically represented in Fig. 6, the instrumentation probe for Raman spectroscopy measurement may be inserted into a flowline of an acid-rich solution. In another embodiment as described in Example 5 and schematically represented in Fig. 7, the instrumentation probe for Raman spectroscopy measurement may be inserted in the output flowline from a salt balancing process vessel. In yet another embodiment as described in Example 6 and schematically represented in Fig. 8, the instrumentation probe for Raman spectroscopy measurement may be inserted in the output flowline from an acid dilution process vessel. In one embodiment as described in Example 7 and schematically represented in Fig. 9, the instrumentation probe for Raman spectroscopy measurement may be submerged in the contents of a salt solution supply vessel. A skilled person in the field of process control will understand that application may involve any of these either by itself or in combination along with the other process control instruments and devices that are integrated with the process control architecture.

Suitable salt solutions include the aqueous precursors of polyamides, which can be charged to batch or continuous polymerizers to carry out condensation reactions to make nylons. Such lists of precursors are well known in the art, and may include those appropriate for manufacturing nylon 6,6, nylon 6,12, nylon 6,10, nylon 4,6, and such like and any copolymers or blends thereof. Inclusion of polymer additives would not be a departure from the envisioned embodiments.

The Raman probe is fixed in a suitable process location as required by the desired embodiment. The illuminating laser is transmitted from the spectrometer via a fiber optic cable through the probe and into the process fluid. The lens of the probe also collects the scattered light. The scattered light is then filtered so that only Raman scattering light is included. This scattered light is then split so that it may be processed into a spectrum of scattered light intensities as a function of wavelength. These data are processed by the computer using spectral processing software such as is familiar to those skilled in the art. Multiple individual spectra can be co-added into a single composite spectrum as required to achieve the desired resolution, with the number of spectra co-added being dependent on the embodiment, configuration, instrument and resolution required. The number of spectra co-added fixes the periodicity of the sampling rate. These Raman spectra are analyzed using the Partial Least Square [or PLS] chemometrics model to give a continuous estimate of real time salt monomeric balance. This monomeric balance estimate then can be used to correct the monomeric balance of the salt for any deviations from target by precise metering of diamine and/or diacid flows to the process.

Dimonomeric polyamides are those which are derived from the condensation polymerization of a dicarboxylic acid and a diamine. Adipic acid and hexamethylenediamine, for example, are commonly polymerized to form nylon 6,6. Copolyamides are often prepared from aqueous solutions or blends of aqueous solutions that contain more than two monomers.

The dicarboxylic acid component is suitably at least one dicarboxylic acid of the molecular formula (I): $HO_2C$-$R1$-$CO_2H$; wherein R1 represents a divalent aliphatic, cycloaliphatic or aromatic radical or a covalent bond. R1 suitably comprises from 2 to 20 carbon atoms, preferably 2 to 12 carbon atoms, more preferably 2 to 10 carbon atoms. R1 may be a linear or branched, preferably linear, alkylene radical comprising 2 to 12 carbon atoms, or 2 to 10 carbon atoms, for example 2, 4, 6 or 8 carbon atoms, an unsubstituted phenylene radical, or an unsubstituted cyclohexylene radical. Optionally, R1 may contain one or more ether groups. Preferably, R1 is an alkylene radical, more preferably a linear alkylene radical, comprising 2 to 12 carbon atoms, or 2 to 10 carbon atoms, for example 2, 4, 6 or 8 carbon atoms.

Examples of suitable diacids include oxalic acid, malonic acid, succinic acid, glutaric acid, pimelic acid, hexane-1,6-dioic acid (adipic acid), octane-1,8-dioic acid (suberic acid), azelaic acid, decane-1,10-dioic acid (sebacic acid), undecanedioic acid, dodecane-1,12-dioic acid, maleic acid, glutaconic acid, traumatic acid, muconic acid, 1,2-cyclohexanedicarboxylic acid, 1,3-cyclohexanedicarboxylic acid, 1,4-cyclohexanedicarboxylic acid, 1,2- or 1,3-phenylenediacetic acids, 1,2- or 1,3-cyclohexane diacetic acids, benzene-1,2-dicarboxylic acid (phthalic acid), benzene-1,3-dicarboxylic acid (isophthalic acid), benzene-1,4-dicarboxylic acid (terephthalic acid), 4,4'-oxybis(benzoic acid), 4,4-benzophenone dicarboxylic acid, 2,6-napthalene dicarboxylic acid, p-t-butyl isophthalic acid and 2,5-furandicarboxylic acid and mixtures thereof. The preferred dicarboxyic acid is adipic acid. These diacids may be supplied in powder form, molten form, as a cake, as a slurry or as aqueous solutions. These diacids may be supplied in a pure form with low levels of impurities or they may be supplied as blended ratios with other diacids or monomers.

The diamine component is suitably at least one diamine of the formula (II): $H_2N$-$R2$-$NH_2$; wherein R2 represents a divalent aliphatic, cycloaliphatic or aromatic radical. R2 suitably comprises from 2 to 20 carbon atoms, preferably 4 to 12 carbon atoms, more preferably 4 to 10 carbon atoms. R2 may be a linear or branched, preferably linear, alkylene radical comprising 4 to 12 carbon atoms, more preferably 4 to 10 carbon atoms, for example 4, 6 or 8 carbon atoms, an unsubstituted phenylene radical, or an unsubstituted cyclohexylene radical. Optionally, R2 may contain one or more ether groups. Preferably, R2 is an alkylene radical, more preferably a linear alkylene radical, comprising 4 to 12 carbon atoms, or 4 to 10 carbon atoms, for example 2, 4, 6 or 8 carbon atoms.

Examples of suitable diamines include ethanol diamine, trimethylene diamine, tetramethylene diamine (putrescine), pentamethylene diamine (cadaverine), hexamethylene diamine, 2-methyl pentamethylene diamine, heptamethylene diamine, 2-methyl hexamethylene diamine, 3 methyl hexamethylene diamine, 2,2-dimethyl pentamethylene diamine, octamethylene diamine, 2,5-dimethyl hexamethylene diamine, nonamethylene diamine, 2,2,4- and 2,4,4-trimethyl hexamethylene diamines, decamethylene diamine, 5-methylnonane diamine, isophorone diamine, undecamethylene diamine, dodecamethylene diamine, 2,2,7,7-tetramethyl octamethylene diamine, bis(p-aminocyclohexyl)methane, bis(aminomethyl)norbornane, C2-C16 aliphatic diamine optionally substituted with one or more C1 to C4 alkyl groups, aliphatic polyether diamines and furanic diamines such as 2,5-bis(aminomethyl)furan, xylylenediamine and mixtures thereof. Hexamethylenediamine is the preferred diamine. It commonly contains a fraction of water to improve handling aspects but it may be supplied in anhydrous form. It may also be supplied to a process in a more dilute form to enhance metering accuracy.

Besides these di-functional monomers, it is sometimes useful to include other monomers. These monomers may include mono-functional carboxylic acids such as formic acid, acetic acid, propionic acid, butyric acid, valeric acid, benzoic acid, caproic acid, enanthic acid, octanoic acid, pelargonic acid, capric acid, undecanoic acid, lauric acid, myristic acid, myristoleic acid, palmitic acid, palmitoleic acid, sapienic acid, stearic acid, oleic acid, elaidic acid, vaccenic acid, linoleic acid, erucic acid and the like. These monomers may also include lactams such as α-acetolactam, α-propiolactam, β-priopiolactam, γ-butyrolactam, δ-valerolactam, γ-valerolactam, caprolactam and the like. These monomers may also include lactones such as α-acetolactone, α-propiolactone, β-priopiolactone, γ-butyrolactone, δ-valerolactone, γ-valerolactone, caprolactone and the like. These monomers may include di-functional alcohols such as monoethylene glycol, diethylene glycol, 1,2-propanediol, 1,3-propanediol, dipropylene glycol, 1,2-butanediol, 1,3-butanediol, 1,4-butanediol, 2,3-butanediol, 1,2-pentanediol, 1,5-pentanediol, etohexadiol, p-menthane-3,8-diol, 2-methyl-2,4-pentandiol, 1,6-hexanediol,, 1,7-heptanediol, 1,8-octanediol and the like. Monomers may include those with one carboxylic acid functional group and one amine functional group such as 6-aminohexanoic acid, 5-aminopentanoic acid, 7-aminoheptanoic acid and the like. Molecules with higher degrees of functionality may be used such as glycerine, trimethylolpropane, triethanolamine and the like. These monomers may also be suitable hydroxylamines such as ethanolamine, diethanolamine, 3-amino-1-propanol, 1-amino-2-propanol, 4-amino-1-butanol, 3-amino-1-butanol, 2-amino-1-butanol, 4-amino-2-butanol, pentanolmaine, hexanolamine and the like. It will be understood that precursor solutions may include any of these monomers without departing from this disclosure.

Suitable examples of the polymer may include, but are not limited to, AABB type polyamide (nylon) resins such as PA22, PA46, PA66, PA77, PA610, PA612, PA1010, PA1212, etc. In the polymer industry, the term "Polyamide" is interchangeably used with "Nylon", both having the same meaning to a person skilled in polymer science. For example, Polyamide 6 (PA6) is also referred to as Nylon 6 (N6). Likewise, Polyamide 66 (PA66) is also referred to as Nylon 66 or nylon-6,6 (N66).

Examples of suitable polyamides include, but are not limited to, polytetramethlyeneadipamide (PA46), polyhexamethlyeneadipamide (PA66), polyhexamethylenedodecamide (PA612), and any combination thereof. Other examples may include, but are not limited to, amorphous and semi-aromatic copolyamides such as poly-hexamethyleneterephthalamide (known as "6T"), poly-hexamethyleneisophthalamide (known as "6I"), 2-methyl-1,5-pentamethyleneterephthalamide (known as "MPMD-T" or "DT"), 2-methyl-1,5-pentamethyleneisophthalamide (known as "MPMD-I" or "DI"), and their combinations such as "66/6T", "66/6I", "6I/6T", "DT/DI", "66/DT/DI", etc.

It should be understood that the concept of producing a polyamide from diamines and diacids also encompasses the concept of other suitable comonomers, such as, aminoacids or lactams. Without limiting the scope, examples of aminoacids can include: 6-aminohaxanoic acid; 7-aminoheptanoic acid; 11-aminoundecanoic acid; 12-aminododecanoic acid. Without limiting the scope of the disclosure, examples of lactams can include: caprolactam, enantholactam; lauryllactam. Suitable feeds for the disclosed process can include mixtures of diamines, diacids, aminoacids and lactams, and can include monofunctional monomers as well as multifunctional monomers.

EXAMPLES

Example 1: PLS Model Development using Raman Spectra

A one liter, jacketed reactor was fabricated with a side port suitable for fixing a 1/2" diameter probe approximately at agitator height or at about the 300 mL fill line. A 1/2" diameter MarqMetrix Performance Ball-probe (available from MarqMetrix Inc., Seattle, Washington) was fixed in place with appropriate process fittings. This probe was connected via 5 meter fiber optic lines to a HyperFlux PRO Plus Raman spectroscopic instrument (available from Tornado Spectral Systems, Toronto, Ontario). This HyperFlux PRO Plus utilized a 785 nm laser with 495 mW power. Each spectral file collected was a co-addition of 25 spectra collected from 750 millisecond exposures.

An initial solution was prepared by carefully weighing components shown per Table 1 and heating the system to about 75°C with agitation. The hexamethylenediamine (HMD) used contained 20% water (table data accounts for this). Components were weighed in using a high precision analytical balance, and the monomeric content was calculated from the gravimetric data. In Table 1, the monomeric balance is characterized as "DE" which represents the difference-of-ends which is calculated by subtracting the milliequivalents of amine groups from the milliequivalents of carboxyl acid groups and normalizing this difference by the total solids weight in solution. Monomeric balance may be assessed in other ways such as by direct titration or via calibrated pH testing. Within Table 1 the gravimetric approach was used.

Multiple spectra were collected for each composition. After collecting the spectra, the solution was modified by carefully weighing in either more adipic acid (AA) or 80% hexamethylenediamine (HMD), and water was added at each step to maintain the concentration.

**Table 1**

| | total solids | AA | HMD | DE | Average |
|---|---|---|---|---|---|
| STEP | [wt %] | [wt %] | [wt %] | [meq/kg] | Temp [°C] |
| **1** | 57.00 | 31.7225 | 25.2775 | -16.08 | 75.4 |
| **2** | 57.00 | 31.7300 | 25.2696 | -11.86 | 74.8 |
| **3** | 57.00 | 31.7331 | 25.2650 | -9.74 | 74.8 |
| **4** | 57.00 | 31.7366 | 25.2605 | -7.53 | 74.8 |

(continued)

|  | total solids | AA | HMD | DE | Average |
| --- | --- | --- | --- | --- | --- |
| STEP | [wt %] | [wt %] | [wt %] | [meq/kg] | Temp [°C] |
| **5** | 57.00 | 31.7471 | 25.2507 | -2.07 | 74.9 |
| **6** | 57.00 | 31.7887 | 25.2113 | 19.83 | 74.9 |
| **7** | 57.00 | 31.6817 | 25.3224 | -39.39 | 75.1 |
| **8** | 57.01 | 31.6992 | 25.3066 | -30.43 | 74.9 |
| **9** | 57.01 | 31.7162 | 25.2918 | -21.87 | 74.8 |
| **10** | 57.01 | 31.7322 | 25.2756 | -13.17 | 74.8 |
| **11** | 57.01 | 31.7460 | 25.2640 | -6.35 | 74.8 |
| **12** | 57.01 | 31.7589 | 25.2506 | 0.81 | 74.8 |

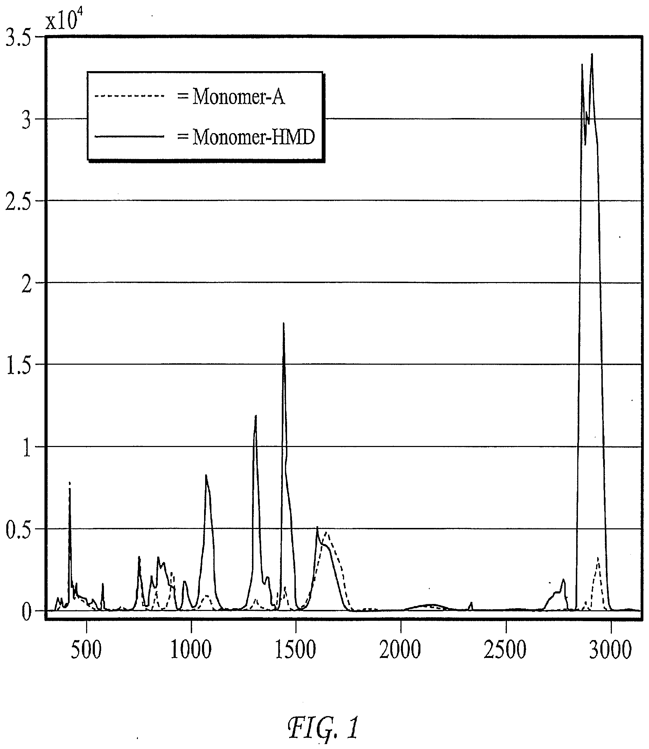

Figure 1 is a representation of an overlay of the Raman spectra of an adipic acid and an HMD solution. In Fig. 1, the X-axis is spectral wavelength in cm-1 and the Y-axis is its corresponding measured intensity. The Fig. 1 data confirmed that there were several wavelength ranges in which the spectra differed, for example, at wavelength regions 800-900 cm-1, ca. 1400 cm-1, 1500 cm-1, ca. 1750 cm-1.

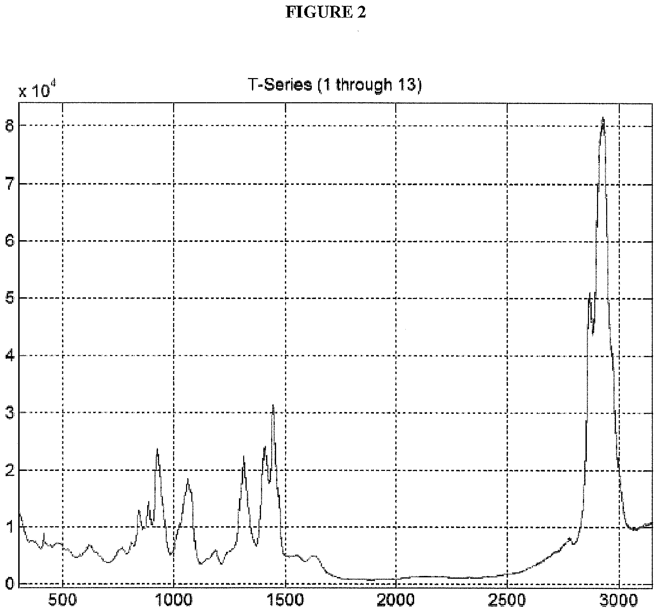

Figure 2 is a representation of measured Raman spectra according to the Table 1 compositions. It was clear that the Fig. 2 spectra showed a different signature compared to the Fig. 1 spectra obtained for individual monomers. Compared to Fig. 1, major peaks were missing, some other peaks appeared, and also, relative peak heights within the spectra shifted in Fig. 2.

The most commonly published Raman studies find distinct differences at specific wavelengths that enable simple models of relative intensity to characterize behaviors. However, this approach was not possible for the Fig. 2 data as the Raman spectra measured for Table 2 compositions did not display distinct differences at specific wavelengths for applying a simple model of relative intensity. The Fig. 2 data revealed no obvious differences at any particular wavelengths.

It was unexpectedly and surprisingly found that, upon applying a partial least square (PLS) analysis for the Fig. 2 Raman spectra data, several wavelength ranges of distinct differences emerged within the PLS model.

After all the spectra were collected, they were analyzed using SimcaP [commercially available from MKS Data Analytics Systems of Malmo, Sweden]. This led to the development of a four component PLS model with excellent predictive capability as represented by an R-squared value of about 0.988.

Table 2 below is a summary of the PLS model result obtained from Raman spectra analysis of the Fig. 2 data.

**Table 2**

| Component | R2X | R2X(cum) | Eigenvalue | R2Y | R2Y(cum) | Q2 | Limit | Q2(cum) |
| --- | --- | --- | --- | --- | --- | --- | --- | --- |
| 0 | Cent. |  |  |  |  |  |  |  |
| 1 | 0.116 | 0.116 | 15.6 | 0.756 | 0.756 | 0.714 | 0 | 0.714 |
| 2 | 0.196 | 0.312 | 26.2 | 0.198 | 0.954 | 0.795 | 0 | 0.941 |
| 3 | 0.134 | 0.446 | 17.9 | 0.0229 | 0.977 | 0.465 | 0 | 0.969 |
| 4 | 0.03... | 0.479 | 4.55 | 0.0167 | 0.993 | 0.615 | 0 | 0.988 |

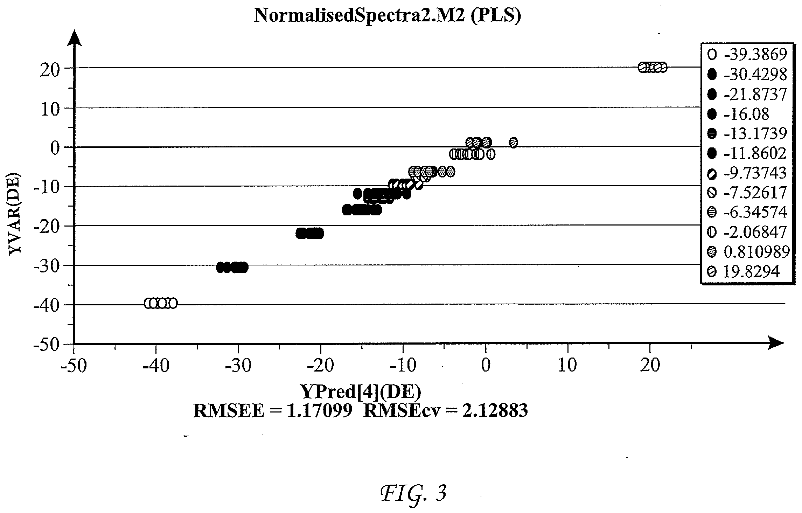

Figure 3 is a representation of the PLS-model to predict DE for the Fig. 2 data. In Fig. 3, the X-axis is PLS model predicted DE values for the Table 1 compositions and the Y-Axis is their corresponding DE values from gravimetric method. For each DE value, a set of multi-point data represents multiple Raman spectra collected and analyzed with excellent reproducibility. The R-squared value for a line [not shown] through the Fig. 3 data points was about 0.988 indicating an excellent model predictability. Thus, Fig. 3 shows the descriptive ability of the PLS model on the subset of spectra that was used to develop it.

Thus, Example 1 demonstrates the practical use of Raman spectroscopy measurements for achieving robust process control to strike a proper monomeric balance ahead of the polyamidation step.

Example 2: PLS Model Predictability for Lab-analyzed Compositions

The model approach according to the Example 1 method was applied for the prediction of process samples that were collected during the operation and analyzed off-line using lab-scale pH/titration analysis.

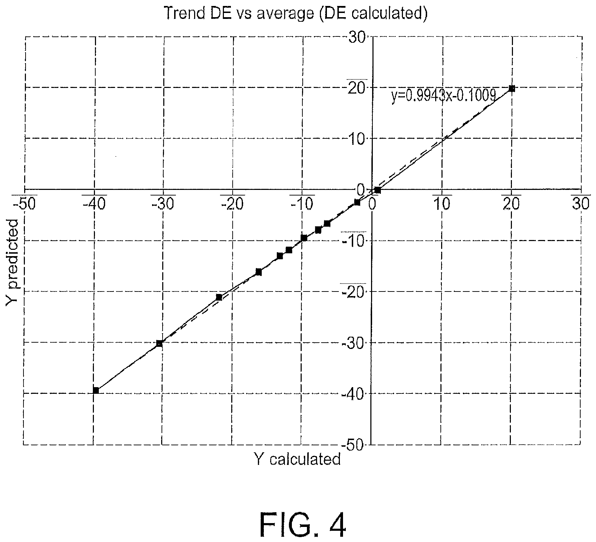

Figure 4 is a parity plot between the DE determined from lab analysis [shown on the X-axis] and predicted DE values [shown on the Y-axis] using the PLS model developed from twelve-point compositional data of Table 1. In Fig. 4, the solid line connects the square data points and the dotted line is its corresponding linear fit with the slope of about 0.994. From the quality of data match, it was clear that the Example 1 model approach obtained excellent predictability, and therefore, can be a useful technique for implementing a robust process control for monomeric balance during polymerization. Thus, Fig. 4 shows how the PLS model, developed from the Example 1 spectra data, performs for process samples of this example.

Example 3: Alternate Statistical Model Development using Raman Spectra

Using the Example 1 spectral data for Table 1 compositions, the below six specific ranges identified as important within the PLS modeling and were selected for meaningful analysis:

Range 1 [R1] : 1639-1762 cm-1
Range 2 [R2] : 2825-2901 cm-1
Range 3 [R3] : 1088-1125 cm-1
Range 4 [R4] : 895-914 cm-1
Range 5 [R5] : 961-985 cm-1
Range 6 [R6] : 825-836 cm-1

The intensities corresponding to the six wavelength ranges in Fig. 2 were integrated for each range. The integral of each range was then used within a multi-variate statistical analysis [MiniTab statistical package]. This analysis resulted in the following multi-variate model for the prediction of DE values:

$$DE = -32845 + 1765\ [R1] - 4.51\ [R2] - 125.9\ [R3] + 1095\ [R4] - 94\ [R5] - 379\ [R6] - 30.2\ [R1]^2 - 16.8\ [R4]^2$$

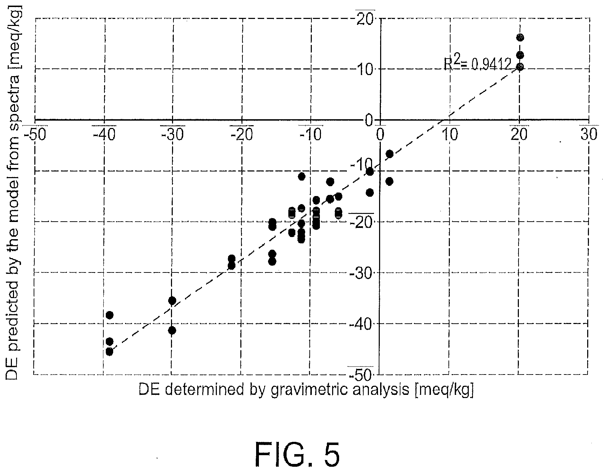

Figure 5 is a representation of the analysis predictability using an alternate modeling approach of this example. In Fig. 5, the DE values of Table 1 data determined via gravimetric analysis are plotted on the X-axis, and the predicted DE values from the above multi-variate model equation are plotted on the Y-axis. The dotted line shown in Fig. 5 obtains the R-squared value of 0.941 and demonstrates reasonably good predictability of the above multi-variate model. For each X-axis data point in Fig. 5, the multi-point data set represents reproducibility of the data.

Thus, Example 3 illustrates the practical use of Raman spectroscopy measurements for achieving robust process control to strike a proper monomeric balance ahead of the polyamidation step.

Example 4: Feed-forward Process Control for Feed Mixture

In this Example, the PLS-model to predict DE approach of Example 1 is applied for determining the monomeric balance, i.e., DE values, in a Feed Forward process control scheme. Figure 6 is a schematic representation of such feed forward control arrangement.

As shown in Fig. 6, a flow line 11 is schematically shown that carries an acid-rich feed mixture [about 2.43:1 AA:HMD on a molar basis] containing about 58% by weight total solids. The feed mixture passes through an inline static mixing device 12. A Raman spectroscopy probe 13 is inserted into the flow line 11 downstream of the static mixing device 12. The Raman spectra for the flowing feed mixture are measured in the Raman spectrometer device 14 and analyzed by computer 15 using the four-component PLS analysis according to Example 1.

The PLS-model to predict DE provides monomeric balance or DE value prediction for the analyzed feed. The resulting signal, shown as hashed lines in Fig. 6, is integrated in a feed-forward manner to meter and control the subsequent addition of 40 wt% aqueous HMD solution through a valved supply line 16 to achieve the desired monomeric balance. The feed with the desired monomeric balance [DE value] is fed forward to a downstream polymerization process.

The static mixing device 12 is optional. If desired, the mixing operation can optionally be accomplished via an agitated vessel or through a pump. Within some process designs it may be found that component miscibility and internal flow turbulence are sufficient such that no additional mixing is required.

Example 5: Feed-back Process Control for Salt Balancing Vessel

In this example, the PLS-model to predict DE approach of Example 1 is applied for determining the monomeric balance, i.e., DE values, in a Feed Back process control scheme. Figure 7 is a schematic representation of such feed-back control arrangement.

As schematically shown in Fig. 7, a baffled, salt balancing process vessel 111 is used for striking a proper monomeric balance before feeding to a downstream polymerization process. Mixing in the salt balancing vessel may be achieved by a motor-driven agitator 112, an external pump circulation loop 113 or a combination of the two as shown in Fig. 7. A heat exchange device 114 in the circulation loop 113 provides the necessary temperature monitoring/control for the salt balancing vessel contents. The salt balancing vessel 111 receives an acid-rich feed [about 2.43:1 AA:HMD on a molar basis] containing about 58wt% total solids, demineralized [DM] water and additional HMD. It is found that any diacid:diamine molar ratio convenient to the process designer may be used without departing from the present disclosure so long as the flow of HMD is in a controllable range, and such considerations are well known in the art. Similarly, other concentrations of the acid-rich feed may be used as convenient without departing from the present disclosure. The salt balancing vessel operates under an inert [nitrogen] blanket maintained in the overhead space.

As shown in Fig. 7, a Raman spectroscopy probe 115 is mounted in the output line 116 from the salt balancing vessel 111. The Raman spectra for the flowing output line mixture are measured by the spectrometer device 117 and analyzed by computer 118 using the four-component PLS analysis according to either Example 1 or Example 2.

The PLS-model to predict DE provides an estimate of monomeric balance or DE value prediction for the analyzed mixture. The resulting signal, shown as hashed lines in Fig. 7, is integrated in a feed-back manner to meter and control addition of 40wt% aqueous HMD solution via inlet line 119 to the pump 121 of the circulation loop 113. The monomeric balance [or DE value] of the salt balancing vessel contents is thereby monitored and adjusted using the feed-back control scheme shown in Fig. 7. The output line feed mixture with the desired monomeric balance is taken to the downstream process.

Though not shown, the four-component PLS analysis signal may be used in feed-back mode to the one or both of the monomer feed controls on the salt balancing vessel. It may also be combined in a feed-forward mode to a trim diamine system as described in Example 4. A combination of the two may also be applied to keep the whole process stable. Alternatively, the signal could be used to control the incoming flow rate of the acid-rich feed to the vessel.

It is also found that the tuning of the HMD control loop can be improved by incorporating secondary instruments [not shown in Fig. 7]. These may include the flow meter on the salt feed or on the salt output line, the level instrument on the salt balancing vessel, valve action indicators or other instruments within the assembly. Such improvements to the tuning do not represent a departure from this example of using Raman instrumentation in a control loop on monomeric balance within a salt balancing vessel.

It is also found that the arrangement of flow connections to the salt balancing vessel are non-limiting to the principle of using Raman instrumentation for control of the monomeric balance. In Figure 7, for example, a single HMD feed is combined with the feed of DM water prior to connection to the acid dissolution vessel. These may be separately connected directly to the vessel without departing from this invention. More than one HMD feeds may also be applied without departing from this example. For example, one or more trim feeds of neat or dilute HMD may be controlled via the Raman instrumentation feedback and one or more coarse HMD feeds may be varied to keep the trim feed in an optimal part of its control range. The Raman probe itself may also be arranged in an independent slip stream, bypass, recirculating sample pot or other arrangement without departing from this example.

It is also found that the design of the mixing system within the salt balancing tank is non-limiting to the principle of using Raman instrumentation for control of the monomeric balance. Different agitator designs may be used. Different baffle designs and arrangements may be used. Jet mixers may be used to supplement or replace the mechanical mixer. Different vessel proportions [LID or aspect ratio] may be used. Many variations are well known in the art. It is desirable that the Raman signal is based on a representative sample of the contents of the salt balancing vessel so that good mixing is required, but the details of how that mixing is achieved can be varied without departing from the present disclosure.

Example 6: Feed-back Process Control for Acid Dissolution Vessel

In this example, the PLS-model to predict DE approach of Example 1 is applied for determining the monomeric balance, i.e., DE values, in a Feed Back process control scheme. Figure 8 is a schematic representation of such feed-back control arrangement.

As schematically shown in Fig. 8, a baffled, acid dissolution process vessel 211 is used for striking a proper monomeric balance before feeding to a downstream polymerization process. Mixing in the acid dissolution vessel 211 may be achieved by a motor-driven agitator 212, an external pump circulation loop 213 or a combination of the two as shown in Fig. 8. A heat exchange device 214 in the circulation loop 213 provides the necessary temperature monitoring/control for the acid dissolution vessel contents. The acid dissolution vessel 211 receives solid adipic acid [AA] feed via a screw conveyer 215 and, demineralized water and HMD via line 216. The acid dissolution vessel 211 may optionally operate under an inert nitrogen blanket (such as nitrogen) maintained in the overhead space or for some compositions it may operate under ambient conditions.

As shown in Fig. 8, a Raman spectroscopy probe 217 is mounted in the circulation loop 213. The Raman spectra for the ciculating feed mixture are measured in the spectrometer device 218 and analyzed by computer 219 using the four-component PLS analysis according to either Example 1 or Example 2.

The PLS-model to predict DE provides an estimate of monomeric balance or DE value prediction for the analyzed mixture. The resulting signal, shown as hashed lines in Fig. 8, is integrated in a feed-back manner to meter and control the diamine feed rate [shown in Fig. 8], or it could optionally be used to vary the screw speed of the acid feeder [not shown in Fig. 8], or it could optionally be used strictly for data collection and estimate of composition for downstream flow control. The output line feed mixture with the desired monomeric balance is taken to the downstream process.

It is also found that the tuning of the HMD control loop can be improved by incorporating secondary instruments [not shown in Fig. 8]. These may include averaging of the AA feed rate from the screw feeder or the flow meter on the salt output line or the level instrument on the mix tank or other instruments within the assembly. Such improvements to the tuning do not represent a departure from this example of using Raman instrumentation in a control loop on monomeric balance within an acid dissolution vessel.

It is also found that the arrangement of flow connections to the acid dissolution vessel are non-limiting to the principle of using Raman instrumentation for control of the monomeric balance. In Figure 8, for example, a single HMD feed is combined with the feed of DM water prior to connection to the acid dissolution vessel. These may be separately connected directly to the vessel without departing from this example. More than one HMD feed may also be applied without departing from this example. For example, one or more trim feeds of neat or dilute HMD may be controlled via the Raman instrumentation feedback and one or more coarse HMD feeds may be varied to keep the trim feed in an optimal part of its control range. The connections of the water and HMD feeds may also be made at other points around the vessel such as to the suction of the recirculation pump or in the heat exchanger loop or another bypass loop or below the liquid level in the vessel. Such commonly known variations of stream arrangements are a matter of convenience to the process designer and do not represent a departure from this example.

It is also found that the design of the mixing system within the acid dissolution tank is non-limiting to the principle of using Raman instrumentation for control of the monomeric balance. Different agitator designs may be used. Different baffle designs and arrangements may be used. Jet mixers may be used. Different vessel proportions [LID or aspect ratio] may be used. Many variations are well known in the art. It is desirable that the Raman signal is based on a representative sample of the contents of the acid dissolution vessel so that good mixing is required, but the details of how that mixing is achieved can be varied without departing from this example.

Example 7: On-line Process Control for Salt Solution Supply Vessel

In this example, the PLS-model to predict DE approach of Example 1 is applied for determining the diamine adjustment that may be made to a salt solution supply vessel that supplies feed to a polymerizer. Figure 9 is a schematic representation of such a process control arrangement.

As schematically shown in Fig. 9, a baffled, salt solution supply vessel 311 is used for fine-tuning of monomeric balance before feeding to the downstream polymerization process. Mixing in the salt solution supply vessel may be achieved by a motor-driven agitator 312 as shown in Fig. 9. The salt solution from upstream salt preparation process feeds to the salt solution supply vessel.

As shown in Fig. 9, a Raman spectroscopy probe 313 is submerged inside the salt solution supply vessel 311. The Raman spectra for the salt solution supply vessel contents are measured in the spectrometer device 314 and analyzed by computer 315 using the four-component PLS analysis according to either Example 1 or Example 2.

The PLS-model to predict DE provides an instant monomeric balance or DE value prediction for the analyzed mixture. The resulting signal, shown as hashed lines in Fig. 9, is integrated to finely adjust the total diamine charge to the salt solution batch in the supply vessel [shown in Fig. 9] which maintains and controls the final monomeric balance desired for the downstream polymerization process. The supply vessel contents are pump transported to the downstream process. Use of diluted HMD as a feed instead of neat HMD is found to provide improvements to control in some cases and that does not represent a departure from this example. Using more than one HMD feed - such as a larger feed for coarse adjustment and a smaller feed for fine adjustment and wherein the multiple feeds may or may not be at the same concentration - is also found to provide improvements to control and is not considered as a departure from this example.

The instrumentation choice used to control the total HMD charge may also be changed without departing from this example. The arrangement of Figure 9 is found to be suitable if this system is used as a fine tuning of the monomeric balance in continuous or semi-continuous mode wherein the HMD flow rate is modulated. Alternative, the arrangement may be used in batch or semi-batch mode wherein a total HMD charge is used. Within that, it is found in some cases that using a small charging pot on a weigh scale is also a suitable instrumentation arrangement. It is beneficial to use estimates of the salt charge - obtained either from totalizing flow meters on the salt feed or via level instrumentation or other means - to tune the HMD charge or flow rate.

Since this is an automated system with fast feedback, it is found that using multiple HMD injections to a batch adjustment is not cumbersome and can be helpful in fine tuning. It is not a departure from this example if either a single HMD addition is used or multiple cycles of HMD addition and spectral sampling are used.

A salt solution supply vessel batch may be monitored in this manner during loading to the polymerization process. The composite co-added spectra from the salt solution supply vessel are used for the desired monomeric balance for the polymerizer operation. Example 8: Control using Process Sampling and Offline Analysis

In the absence of the disclosed online control scheme using Raman spectra analysis as described in Examples 4 through 7, a feed sample is drawn from a sampling point; the location of which is schematically shown in either of Figs. 6, 7, 8 or 9. In each case, the collected feed sample is needed to be properly conditioned, handled, transported to the lab and analyzed offline. From the lab analysis, such as pH/titration methods, the monomeric balance is determined. The deviation from the target monomeric balance then accounts for any necessary adjustments to feed streams and other feed devices. There is a time delay of several hours from the time the process sample is taken and by the time lab analysis becomes available for control. This time lag between the sampling and monomeric balance determination is not desirable from a viewpoint of maintaining steady-state downstream process condition. In this approach, the offline analysis constantly struggles to catch-up with the real-time process condition. This becomes difficult, especially, when the process goes through any step-change transients or unsteady conditions. The overall polyamidation process suffers from delayed process adjustments and off-target production with increased material losses.

## Claims

1. A process control method for measuring and controlling diacid-diamine balance of a polyamide precursor solution comprising:

   a) creating a predictive model of Raman spectra for diacid-diamine balance by:

   i) acquiring Raman spectra for a series of polyamide precursor solutions having different known molar ratios of diacid:diamine;
   ii) modeling the relationship between the known molar ratios and the acquired Raman spectra;

   b) applying the model of (a)(ii) to an observed Raman spectrum for a polyamide precursor solution in which the diacid:diamine balance is unknown; and
   c) determining the diacid:diamine balance of the solution (b).

2. The process control method of claim 1 further comprising comparing the diacid:diamine balance determined in step (c) with a setpoint.

3. The process control method of claim 2 further comprising adjusting at least one process variable in response to the comparison of the determined diacid:diamine balance with the setpoint.

4. The method of claim 3. wherein the process variable that is adjusted is selected from the group consisting of: flow rate of dicarboxylic acid or of a dicarboxylic acid-rich feed stream, flow rate of diamine or of a diamine-rich feed stream, residence time, and flow rate of additive.

5. The method of any one of the preceding claims wherein the modeling a (ii) comprises partial least squares statistical analysis.

6. The method of any one of the preceding claims, wherein the polyamide precursor solution comprises dicarboxylic acid, diamine and water.

7. The method of any one of the preceding claims, wherein the dicarboxylic acid comprises adipic acid.

8. The method of any one of the preceding claims, wherein the diamine comprises hexamethylenediamine.

9. A process of preparing a polyamide comprising the process control method of any one of the preceding claims.

**10.** The process of claim 9, wherein the polyamide is nylon 6,6.


**Patentansprüche**

**1.** Prozesskontrollverfahren zum Messen und Kontrollieren des Verhältnisses von Disäure zu Diamin in einer Polyamidvorläuferlösung, umfassend:

a) Erstellung eines Vorhersagemodells von Raman-Spektren für das Verhältnis von Disäure und Diamin durch:

i) Erwerb von Raman-Spektren für eine Reihe von Polyamid-Vorläuferlösungen mit verschiedenen bekannten molaren Verhältnissen von Disäure zu Diamin;
ii) Modellierung der Beziehung zwischen den bekannten Molverhältnissen und den erfassten Raman-Spektren;

b) Anwendung des Modells von (a)(ii) auf ein beobachtetes Raman-Spektrum für eine Polyamid-Vorläuferlösung, in der das Verhältnis von Disäure zu Diamin unbekannt ist; und
c) Bestimmung des Verhältnisses von Disäure zu Diamin in der Lösung (b).

**2.** Prozesskontrollverfahren nach Anspruch 1, weiter umfassend den Vergleich des in Schritt (c) bestimmten Verhältnisses von Disäure zu Diamin mit einem Vorgabewert.

**3.** Prozesskontrollverfahren nach Anspruch 2, weiter umfassend das Einstellen mindestens einer Prozessvariablen als Reaktion auf den Vergleich des ermittelten Verhältnisses von Disäure zu Diamin mit dem Vorgabewert.

**4.** Verfahren nach Anspruch 3, wobei die Prozessvariable, die eingestellt wird, ausgewählt ist aus der Gruppe bestehend aus: Durchflussrate der Dicarbonsäure oder eines dicarbonsäurereichen Zufuhrstroms, Durchflussrate des Diamins oder eines diaminreichen Zufuhrstroms, Verweilzeit und Durchflussrate eines Additivs.

**5.** Verfahren nach einem der vorstehenden Ansprüche, wobei die Modellierung a (ii) eine statistische Analyse der partiellen kleinsten Quadrate umfasst.

**6.** Verfahren nach einem der vorstehenden Ansprüche, wobei die Polyamid-Vorläuferlösung Dicarbonsäure, Diamin und Wasser enthält.

**7.** Verfahren nach einem der vorstehenden Ansprüche, wobei die Dicarbonsäure Adipinsäure umfasst.

**8.** Verfahren nach einem der vorstehenden Ansprüche, wobei das Diamin Hexamethylendiamin umfasst.

**9.** Verfahren zur Herstellung eines Polyamids, umfassend das Prozesskontrollverfahren nach einem der vorstehenden Ansprüche.

**10.** Verfahren nach Anspruch 9, wobei das Polyamid Nylon 6,6 ist.


**Revendications**

**1.** Procédé de commande de processus destiné à mesurer et à commander l'équilibre diacide/diamine d'une solution de précurseur de polyamide comprenant :

a) la création d'un modèle prédictif des spectres Raman pour l'équilibre diacide/diamine par :

i) l'obtention des spectres Raman pour un ensemble de solutions de précurseur de polyamide présentant différentes rapports molaires connus de diacide/diamine ;
ii) la modélisation de la relation entre les rapports molaires connus et les spectres Raman obtenus ;

b) l'application du modèle de (a)(ii) à un spectre Raman observé pour une solution de précurseur de polyamide dans laquelle l'équilibre diacide/diamine est inconnu ; et

c) la détermination de l'équilibre diacide/diamine de la solution (b).

2. Procédé de commande de processus selon la revendication 1 comprenant en outre la comparaison de l'équilibre diacide/diamine déterminé à l'étape (c) avec une valeur de consigne.

3. Procédé de commande de processus selon la revendication 2 comprenant en outre le réglage d'au moins une variable de processus en réponse à la comparaison de l'équilibre diacide/diamine déterminé avec la valeur de consigne.

4. Procédé selon la revendication 3, dans lequel la variable de processus qui est réglée est choisie parmi le groupe consistant en : débit d'acide dicarboxylique ou d'un flux d'alimentation riche en acide dicarboxylique, débit de diamine ou d'un flux d'alimentation riche en diamine, temps de séjour et débit d'un additif.

5. Procédé selon l'une quelconque des revendications précédentes dans lequel la modélisation a (ii) comprend une analyse statistique des moindres carrés partiels.

6. Procédé selon l'une quelconque des revendications précédentes, dans lequel la solution de précurseur de polyamide comprend de l'acide dicarboxylique, de la diamine et de l'eau.

7. Procédé selon l'une quelconque des revendications précédentes, dans lequel l'acide dicarboxylique comprend de l'acide adipique.

8. Procédé selon l'une quelconque des revendications précédentes, dans lequel la diamine comprend de l'hexaméthylènediamine.

9. Processus de préparation d'un polyamide comprenant le procédé de commande de processus selon l'une quelconque des revendications précédentes.

10. Processus selon la revendication 9, dans lequel le polyamide est du nylon 6,6.

*FIG. 1*

**FIGURE 2**

T-Series (1 through 13)

**NormalisedSpectra2.M2 (PLS)**

Legend:
- ○ -39.3869
- ● -30.4298
- ● -21.8737
- ● -16.08
- ◒ -13.1739
- ● -11.8602
- ◑ -9.73743
- ◔ -7.52617
- ◉ -6.34574
- ◍ -2.06847
- ◉ 0.810989
- ◎ 19.8294

**YPred[4](DE)**
**RMSEE = 1.17099  RMSEcv = 2.12883**

*FIG. 3*

Trend DE vs average (DE calculated)

FIG. 4

FIG. 5

**FIG. 6**

FIG. 7

HMD

DEMIN

N2

AA

*215*

*211*

Vent

*216*

Level

M

*213*

*214*  Heat
Exchanger

*212*

*217*

*219*  Computer

*218*  Spectrometer

I
P

SAMPLING POINT

TO PROCESS

# FIG. 8

FIG. 9

## REFERENCES CITED IN THE DESCRIPTION

**Patent documents cited in the description**

- US 3730685 A
- US 4213884 A
- US 4251653 A
- US 4442260 A
- US 5801278 A
- US 9561999 B
- WO 2014179057 A
- WO 2014179058 A
- WO 2014179060 A
- WO 2014179061 A
- WO 2014179062 A
- WO 2014179064 A
- WO 2014179065 A
- WO 2014179066 A
- US 8772439 B
- US 8802810 B
- US 4233234 A
- US 2130947 A
- US 5532487 A
- US 6169162 B
- US 6696544 B
- US 6995233 B
- US 4620284 A, Schnell